# EUROPEAN PATENT APPLICATION

(11) **EP 1 275 738 A1**
(43) Date of publication of application: **15.01.2003**
(21) Application number: 02015233.6
(22) Date of filing: 09.07.2002
(51) Int. Cl.: C12Q 1/68, C12N 15/10

(54) **Method for random cDNA synthesis and amplification**

(30) Priority: 11.07.2001 EP 01115787
(71) Applicant: Roche Diagnostics GmbH, 68305 Mannheim (DE); F. HOFFMANN-LA ROCHE AG, 4070 Basel (CH)
(72) Inventor: Frey, Bruno, Dr., 82377 Penzberg (DE); Brehm, Ursula, 82166 Graefelfing (DE); Eichner, Cornelia, 82377 Penzberg (DE); Wartbichler, Regina, 82404 Sindelsdorf (DE)

(57) **Abstract**

The new invention is directed to a method for amplification of a pool of RNA sequences, comprising (I) synthesis of total first strand cDNA with a first primer comprising a first segment located at the 3' terminal part of said oligonucleotide which is capable of hybridizing to substantially all RNAs contained in the RNA pool and a second segment, said second segment being located more proximal to the 5' end of said oligonucleotide, said second segment being capable of serving as a primer binding side for another nucleic acid amplification primer by itself and (II) synthesis of total second strand cDNA with a second primer, said second primer comprising a first randomized segment located at the 3' end of said primer and a second segment which is located more proximal to the 5' end of said oligonucleotide, said second segment being capable of serving as a primer binding side by itself for a nucleic acid amplification primer such that a double stranded cDNA is generated.

## Description

The present invention relates to the field of amplifying a whole population of nucleic acids. More specifically, the present invention relates to the field of random cDNA amplification. The new method can be used in order to analyze the expression status of known genes or identify differentially expressed genes from very small numbers of cell populations.

### Prior art

Amplification of total nucleic acids is required for many different experimental set-ups for diagnostic an research purposes. Total nucleic acids amplification has been described both for genomic DNA and cDNA.

Total amplification of DNA is usually called whole genome amplification. This type of analysis can be used for diagnostic of inherited diseases, for example during a pre-implantation diagnostics of blastomere biopsy (Kristjansson, K., et al., Nat Genet (1994) 19-23). Another important application is an analysis of microsatellite instability as a prognostic marker for sporadic colorectal carcinoma (Thibodeau, S. N., et al., Science (1993) 816-9).

Amplification of total cDNA is of major interest in the context of gene expression analysis especially on multi-hybridization-event-based assay systems, like hybridization on DNA Arrays (Schena, M., et al., Science (1995) 467-70). Conventional methods like Northern Blots, RT-PCR, etc., have the disadvantage of measuring only one or a few messages simultaneously. However, simultaneous expression analysis of a plurality of mRNAs allows for the rapid identification of differentially expressed genes, especially if whole RNA populations from different sources like e.g. tumor cells and normal cells are compared. Even more important, a simultaneous expression analysis is necessary for the analysis and of diseases which are due to an altered expression of a multiplicity of different genes.

Among the several methods that have been described in the art for PCR based random nucleic acid amplification, the following are of major importance:

### a) Whole genome amplification (WGA):

Starting from genomic DNA or reverse transcribed total cDNA, a first PCR is performed with completely randomized primers, resulting in an amplification of the complete population. Subsequently, genes of interest are amplified using specific primers in a second PCR amplification reaction. However, in order to have each gene or mRNA represented in a statistically manner corresponding to its actual abundancy, and further in order to exclude preparation artifacts, WGA can only be performed on genomic DNA or total cDNA that has been obtained from a large number of cells. A specific embodiment of whole genome amplification is carried out using a mixture of a polymerase with and a second polymerase without 3' 5' exonuclease proofreading activity (EP 0 957 177).

### b) RNA fingerprint (AFLP: amplification fragment length polymorphism)

Methods using short arbitrary or partially arbitrary primers are used on a DNA basis for finger printing analysis in order to discriminate complex genomes (Welsh, J. McClelland, M., Nucleic Acids Res (1990) 7213-8). No further sequence information is required in advance. Usually, amplification in this case starts with low stringency, but is increased during the later cycles. In many embodiments, the amplified products are subjected to a specific restriction enzyme digest. For expression analysis these methods have been adapted to analyze cDNAs (Money, T., et al., Nucleic Acids Res (1996) 2616-7).

In an alternative approach, prior to the amplification step, the double-stranded cDNA obtained with an Oligo-dT primer is digested with a restriction endonuclease. Subsequently, suitable adapters with arbitrary sequences and amplification primer binding sites are ligated to the generated cDNA fragments such that amplification of a subset of cDNAs can be carried out (WO 98/51789).

### c) Differential display

For mRNA differential display, reversed transcribed cDNA is amplified using only partially randomized primers. In this case, the primer used for both reverse transcription and subsequent amplification consists of a 5' segment and which specifically binds to a sub-population of RNA and a second segment located 3' with an arbitrary sequence. For example, the 5' region may be Oligo-dT in order to selectively enrich polyadenylated mRNA. The second primer also comprises an arbitrary sequence.

However, during PCR, this primer design only results in amplification of a specific sub-population of the original RNA-Pool (US 5,262,311).

RNA fingerprint and differential display analyses however have one major drawback: due to the usage of not completely randomized primer, both method are only detecting a subpopulation of the total RNA pool present in a sample.

### d) Smart probe amplification (US 5,962,272)

Most mRNA expression studies known in the art which are based on RT-PCR have the disadvantage that cDNA synthesis is very often incomplete and therefore the primary single-stranded cDNA products very often lack the respective 3' end complementary 5' end of the corresponding cDNA. Therefore, in an alternative approach, first strand cDNA synthesis is performed by an RNAseH lacking reverse Transcriptase which results in an addition of several C-residues at the very end of the cDNA synthesis after the Reverse Transcriptase passed the 5'-Cap-side of the reverse transcribed RNA. A second "template switching" oligonucleotide comprising an amplification primer binding side and a G-stretch complementary to the C-residues at the end of the first strand cDNA. Upon appropriate hybridization between first strand cDNA and the template switching Oligonucleotide, elongation further occurs in both directions such that a double-stranded cDNA is generated. The double-stranded cDNA is then subsequently amplified by suitable PCR amplification primers. This results in an exclusive amplification of any full-length cDNA present in the original population.

However, this selection for full-length cDNA has the disadvantage that not each type of mRNA is represented to the same extend in the amplified pool as compared to its abundance in the original mRNA population.

### e) SABRE: Selective application via biotin and restriction mediated enrichment (Lavery, D. J., et al., Proc Natl Acad Sci U S A (1997) 6831-6)

This method is based on separate amplification of two different populations of cDNAs using different amplification primers some of which are labeled and/or contain a restriction site. The two amplified populations are subsequently hybridized. Due to the specific primer design and an enrichment step via affinity binding, sequences may be isolated which differ with regard to their abundance in the different original population and thus, are differentially expressed in the original pools of RNA.

However, since this method requires a restriction enzyme digest, several important RNAs of interest may escape analysis.

Summarizing all the methods cited above have one or several major drawbacks as follows:
- Inconvenience
- Tendency for a selective enrichment of sub-population of cDNAs
- Low sensitivity due to the requirement of large amounts of starting material.

### Brief description of the invention

However, there is a need in the art for methods of generating populations of amplified cDNA reflecting true levels of expression of each corresponding RNA, which may become derived from a number of cells like biopsy material. Such a pool of amplified cDNA for example may be used for hybridization experiments in reverse Dot Blot analysis or in more complex systems like hybridization on MicroArrays that carry immobilized nucleic acids probes. An example for such an array system is given in WO 01/11082.

Accordingly, in a first aspect, the new invention is directed to a method for amplification of a pool of RNA sequences, comprising:
a) synthesis of total first strand cDNA with a first primer, said first primer being an extendible oligonucleotide comprising a first segment located at the 3' terminal part of said oligonucleotide which is capable of hybridizing to substantially all RNAs contained in the RNA pool and a second segment, said second segment being located more proximal to the 5' end of said oligonucleotide as compared to said first segment, said second segment being capable of serving as a primer binding side for another nucleic acid amplification primer by itself
b) synthesis of total second strand cDNA with a second primer, said second primer being an extendible oligonucleotide comprising a first randomized segment located at the 3' part of said primer and a second segment which is located more proximal to the 5' end of said oligonucleotide as compared to said first segment, said second segment being capable of serving as a primer binding side by itseelf for a nucleic acid amplification primer such that a double stranded cDNA is generated
c) amplification of said double stranded cDNA

In a specific embodiment said first segment of said first primer is an oligo-dT sequence or mainly oligo-dT sequence interrupted with a few universal base nucleotides.

The pool of RNA sequences to be amplified may be either total cellular RNA or mRNA.

Depending on the RNA pool to be amplified, the randomized region of said second extendible primer may differ in its length. Preferably, the length of this region is between 4 to 30 and most preferably between 8- 12 nucleotides.

For subsequent expression studies, the amplified cDNA may become *in vitro* transcribed in an additional step d). In order to enable transcription, the first extendible primer preferably comprises a promoter for *in vitro* transcription.

In a specific embodiment, the products of the *in vitro* transcription may be subjected to a second amplification reaction. In order to achieve this, steps a)-d) are repeated at least once.

For expression profiling analysis, it is advantageous, if a detectable label is incorporated either during or subsequent to the amplification reaction, or, alternatively, during or subsequent to the *in vitro* transcription reaction. In addition, the invention is also directed to a pool of nucleic acids, which has become labeled according to such a method.

Another aspect of the invention is directed to a method for expression profiling, wherein such a pool of labeled nucleic acids is hybridized to a plurality of nucleic acid probes, said probes being immobilized on a surface. For example, the plurality of nucleic acid probes may be immobilized on a solid support (e.g. glass plate).

In a specific embodiment said labeled pool of nucleic acids is cut into fragments prior to hybridization. It has turned out to be advantageous, if the generated fragments are between 50 and 200 nucleotide residues in length.

### Description of the Figures

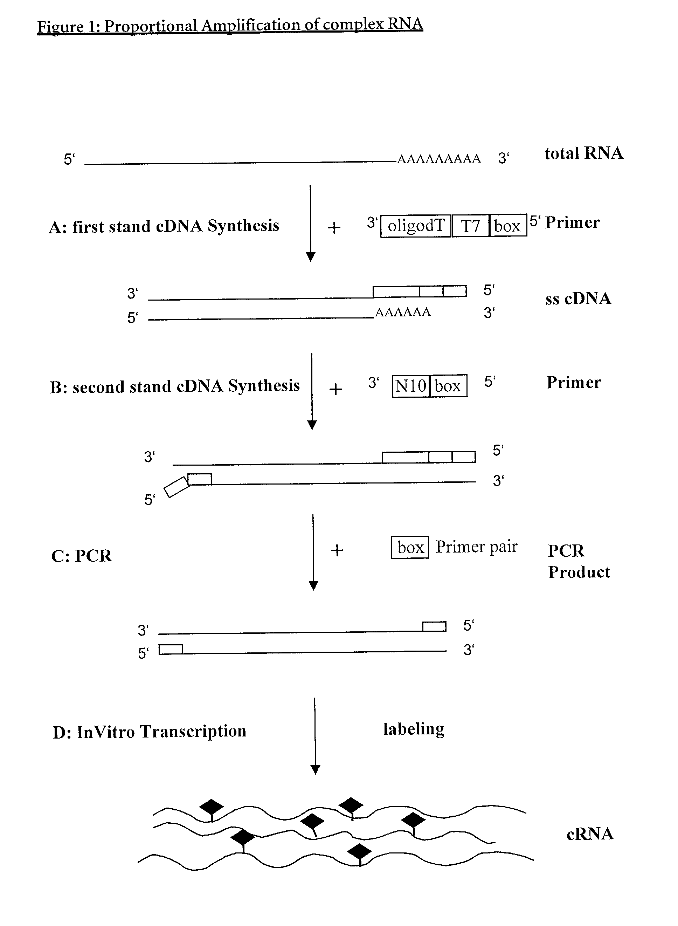

**Fig. 1** shows a schematic drawing of the amplification method according to the invention showing the structure of oligonucleotides according which are used for first and second strand cDNA synthesis.
   "OligodT" indicates a stretch of dT residues, "T7" indicates a T7-promoter sequence, and "box" indicates the sequence of a primer binding site, within the first and second strand synthesis primers according to the invention.
   In the specific embodiment shown in this figure, only one primer is used for the amplification step.

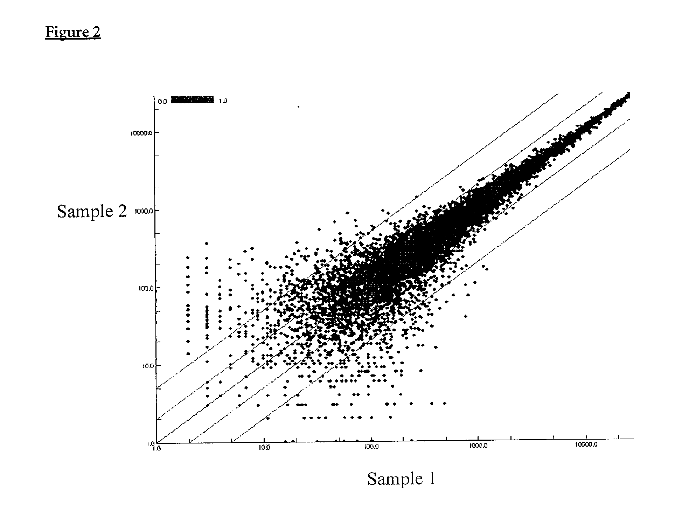

**Fig. 2** shows a scatter blot of the signal intensities of multiple mRNA species detected in two independent experiments according to example 6, indicating the reproducibility of the claimed method.

### Detailed descrition of the invention

For certain expressions used in the context of this invention, the following definitions apply:
The term "pool of RNA sequences" shall mean a total population of RNA molecules which is present in a sample, wherein these RNA molecules have a plurality of many different sequences. Depending on the mode of isolation, a pool of RNA sequences may be total cellular RNA or total cellular mRNA (which is total cellular poly-A RNA).
The term "amplification of a pool of RNA sequences" shall mean a method for reverse transcribing all members of RNA molecules into cDNA and subsequently amplifying the cDNA molecules by means of PCR. It may also include additional amplification by means of *in vitro* transcription.
The term " oligonucleotide" shall mean any kind of a single stranded Oligonucleotide chain including but not limited to Oligo-2'-Deoxy-Nucleotides, wherein the residues are connected by a 5'-3' linkage. It also includes single stranded molecules, which completely consist of or at least comprise one ore more Deoxynucleotde derivatives like e.g. Phosphothiates, universal base . In addition, it shall mean Peptide Nucleic Aids (Nielsen, P. E., et al., Science (1991) 1497-500) or any other polymer capable of hybridizing to polynucleotided targets. Oligonucleotides can be chemically synthesized de novo by methods well known in the art (Matteucci, M. D. Caruthers, M. H., J. Am. Chem. Soc. (1981) 3185-3191).
The term "primer" shall mean any kind of molecule which has a sequence complementary or at least substantially complementary to a target sequence over a stretch of at least 10 residues such that the primer is capable of binding specifically to the (single stranded) target sequence under common buffer conditions known in the art. In addition, the primer has a free 3' end, such that in case the primer is bound to a target sequence forming a partially double stranded hybrid, any kind of primer extension reaction catalyzed by a DNA polymerase including but not limited to enzymes like Thermus aquaticus polymerase, Klenow polymerase and reverse Transcriptase can occur.
The term "segment" of an oligonucleotide shall mean a continuous stretch of nucleotide residues of an oligonucleotide..
The term "terminal part" of an oligonucleotide shall mean a continuous stretch of an nucleotide residues which includes either the 3' or the 5' terminal nucleotide residue.
The term "proximal to an end of an oligonucleotide" shall mean a closer position of a segment to either the 5' end or the 3' end of the oligonucleotide as compared to another segment.
The term "downstream of" shall mean more proximal to the 3' end of a nucleic acid molecule.
The term "upstream of" shall mean more proximal to the 5' end of a nucleic acid molecule.
The term "amplification of an RNA sequence" shall mean reverse transcription of an RNA or a pool of RNA molecules to a double stranded cDNA followed by amplification of the cDNA by means of PCR.
The term "randomized sequence" of an oligonucleotide or oligonucleotide segment or nucleotide shall mean that during chemical synthesis of the respective oligonucleotide by means known in the art, at one or more certain position different residues may become incorporated or the randomized sequence consist of a universal base.
The term "completely randomized" shall mean that A, G, T, or C residues are incorporated arbitrarily , each with an overall equal probability of about 25%. Alternatively the complete sequence may consist of a stretch of universal base residues.
The term "universal base residue" itself shall mean any kind of nucleotide derivative which as part of a polynucleotide chain is capable to form base pairs with A,G, C, and U or T residues.

First strand cDNA synthesis according to the invention is done with a particular oligonucleotide serving as a primer. This first primer according to the inventive method is an extendible oligonucleotide comprising a first segment located at the 3' terminal end of said oligonucleotide which is capable of hybridizing to substantially all RNAs contained in the RNA pool and a second segment, said second segment being located more proximal to the 5' end of said oligonucleotide as compared to said first segment, said second segment being capable of serving as a primer binding site for another nucleic acid amplification primer by itself. For initiation of first strand cDNA synthesis, only the first segment of the primer specifically hybridizes to the pool of RNA molecules to become amplified.

The first segment of the first strand oligonucleotide may consist of a completely or at least partially randomized sequence. For complete amplification of a pool of RNA sequences, it has been found to be advantageous, if this randomized sequence is between 6 and 20 nucleotides in length and most advantageously 8-12 nucleotides in length.

In an alternative embodiment said first segment of said first primer may comprise an oligo-dT sequence, which is capable of hybridizing to substantially all mRNA species. Preferably, the oligo-dT sequence is at least 15 nucleotides in length.

In an alternative embodiment, said first segment of said first primer may comprise beside Thymidin (dMTP) also universal base nucleotides. Oligonucleotides comprising such a sequence do have the advantage that binding to the target RNA necessarily only occurs in anti-parallel orientation.

In one embodiment, the complete segment may only consist of such an oligo-dT sequence or a combination of Thymidin and universal base nucleotides. It is possible and within the scope of the invention, however, that in addition to the sequence above further residues are located at the 3' end of the segment which allow for a hybridization to all RNA molecules found in the pool to become reverse transcribed. Respective embodiments, for example are additional randomized residues.

In another preferred embodiment, in addition to the oligo-dT stretch or the combination of Thymidin and universal base nucleotides, the first segment may comprise a ...VN-3' terminus, wherein V represents a position randomized with respect to A, G and C residues and N represents a position randomized for A, G, C and T. Alternatively, the sequence may comprise a ....VX-3' terminus, wherein V represents a position randomized with respect to A,G and C residues and X represents a universal base residue. Oligonucleotides comprising such a sequence do have the advantage that binding to the target mRNA necessarily occurs at a defined position at the 5' end of the poly-A tail.

The second segment of the first strand oligonucleotide comprises a definite sequence which is not randomized with a preferable length of 10 - 30 residues. The sequence can be designed arbitrarily, but in such a way that using this segment as a primer binding site by itself in the subsequent amplification step may be performed.

An example for an oligonucleotide suitable for first strand cDNA synthesis of a pool of RNA is given in Seq. Id. No. 4, consisting of a 5' 27 residue amplification primer binding site, a 18mer oligo-dT stretch and 2 randomized residues at its 3' end.

For any kind of special purpose, the first strand cDNA synthesis oligonucleotide may also comprise additional segments either between said first segment and said second segment disclosed above or 5' from said second segment serving as an amplification primer binding site.

In a specific embodiment, an additional segment between said first and said second segment constitute a promoter sequence which may be used for *in vitro* transcription in order to generate a high yield of *in vitro* transcribed RNA representing the original pool of reverse transcribed RNA. Due to its location between the other two segments, the promoter will in any case become amplified in the subsequent PCR amplification step.

An example for an oligonucleotide according to the invention enabling transcription of the cDNA synthesized according to the invention is Seq. Id. No. 1. It is representing a 75 mer with a sequence according to Seq. Id. No. 4, with an internal 28bp T7-promoter sequence ranging from nucleotide position Nr. 22-50.

Second strand cDNA synthesis according to the invention is done with another particular oligonucleotide serving as second strand primer. This second primer is an extendible oligonucleotide comprising a first randomized segment located at the 3' end of said primer and a second segment which is located more proximal to the 5' end of said oligonucleotide as compared to said first segment, said second segment being capable of serving as a primer binding side by itself for a nucleic acid amplification primer such that a double stranded cDNA is generated.

The first segment of the second strand oligonucleotide is an at least partially randomized sequence, which however, is preferably completely randomized. Depending on the RNA pool to be amplified, the length of this randomized segment may differ but should not be smaller than 4 and not exceed 30 residues. Preferably, the length of this region is between 8 to 12 and most preferably 10 nucleotides.

The second segment of the second strand oligonucleotide similar to the second sequence of the first strand oligonucleotide comprises an arbitrarily chosen but definite sequence which is not randomized with a preferable length of 10- 30 residues. However, the sequence again is chosen in such a way, that an optimized amplification step using this segment as a second amplification primer binding site may be performed. This includes that the binding sites for both amplification primers, one being complementary to the second segment of the first oligonucleotide and the second being complementary to the second segment of the second oligonucleotide confer about the same annealing temperature.

In a preferred embodiment, the amplification primer binding sites of said first and second oligonucleotides disclosed above are identical such that in the subsequent amplification step only one amplification primer is needed.

An example for a second oligonucleotide according to the invention suitable for second strand cDNA synthesis of a pool of RNA is given in Seq. Id. No. 2, which is a 31mer consisting of a 5' 21 residue amplification primer binding site, and 10 completely randomized residues at its 3' end.

Similar to the first oligonucleotide the second strand cDNA synthesis oligonucleotide may also comprise additional segments either between said first segment and said second segment disclosed above or 5' from said second segment serving as an amplification primer binding site.

The pool of RNA sequences to be amplified may be either derived from total cellular RNA or mRNA which may have been purified by any kind of purification known in the art. This includes but is not limited to purification protocols using Guanidinium and phenol (Chomczynski, P. Sacchi, N., Anal Biochem (1987) 156-9) or quick chromatographic procedures like High Pure spin columns Roche Diagnostics)

Synthesis of cDNA can be performed according to different protocols, wherein first strand and second strand synthesis are either performed by two different enzymes or by using the same enzyme for both steps. For the first strand synthesis, any kind or RNA dependent DNA Polymerases (reverse transcriptase) may be used, e.g. AMV Reverse Tanscriptase, MMLV Reverse Transcriptase or mutants thereof or thermoactive DNA polymerases like C.therm or Tth(Roche Diagnostics). Subsequently, the RNA/cDNA hybrid is subjected to thermal denaturation or alternatively, the RNA template within the hybrid may be digested by RnaseH. Second strand synthesis may then be performed with any kind of DNA dependent DNA Polymerase, e.g. Klenow DNA Polymerase or Taq DNA Polymerase. For an approach where first and second strand synthesis is done with one enzyme, Thermus thermophilus or C.Therm (Roche Diagnostics) may be used.

It has been proven to be advantageous, if the double stranded cDNA is subsequently being purified prior to amplification. Purification may be achieved by means of conventional precipitation, Phenol/Chloroform extraction, using Sephadex spin coloumns, HighPure spin coloumns (Roche Diagnostics) or any other method known in the art.

Amplification of the double stranded cDNA is preferably performed by means of PCR using a thermostable DNA polymerase and two amplification primers which are completely or at least substantially complementary to the primer binding sites introduced by the first and the second oligonucleotide, respectively. Due to the general knowledge on PCR, a person skilled in the art will be capable of establishing suitable PCR conditions for effective high yield amplification through routine development procedures. In addition, other amplification methods, like e.g. NASBA (Kievits, T., et al., J Virol Methods (1991) 273-86) may also be applied.

For subsequent expression studies, the amplified cDNA may become *in vitro* transcribed in an additional step by methods well known in the art (Milligan, J. F., et al., Nucleic Acids Res (1987) 8783-98). In order to enable transcription, the first extendible primer comprises a promoter for *in vitro* transcription. It is essential that the promoter is located downstream of the segment which by itself is capable of serving as a primer binding site for nucleic acid amplification primers. In addition it is required that the promoter is located upstream of the segment which is capable of hybridizing to substantially all RNAs contained in the pool to become amplified. In other words: the promoter is preferably located between said first and said second segment.

For high yield transcription, different procaryotic promoters like the Bacteriophage promoters SP6, T3, and T7 may be used. Best results, however are usually obtained with the T7- promoter.

In case the pool of RNA sequences has been derived from a very limited number of cells it may be advantageous to subject the products obtained by PCR and facultative subsequent *in vitro* transcription to additional steps of amplification. For example, the pool of PCR products obtained might be subjected to a second PCR using the same PCR amplification primers as before. In another embodiment, nested primers might be used in case the oligonucleotides used for first and second strand cDNA synthesis have been designed appropriately.

In case the PCR product has been *in vitro* transcribed, it is necessary for additional amplification to repeat first strand cDNA synthesis, second strand cDNA synthesis and the subsequent PCR reaction again. Due to the design of primers according to the invention, however, it is possible to use the same primers for all these reaction as disclosed above. If desired, the PCR product may then be subjected to a second *in vitro* transcription reaction in order to generate RNA.

For expression profiling analysis, it is usually also desired to incorporate a detectable label during the one or other step of the protocol disclosed above. For example, the label may become incorporated during the PCR amplification reaction by using either a pre-labeled primer or, alternatively, a pre-labeled Deoxynucleoside-triphosphate which both become incorporated into the PCR product. Alternatively, post-labeling can be performed by any kind of method known in the art, e.g. by using a ChemLink reagent (Roche Diagnostics).

Similarly, *in vitro* transcribed RNAs may be labeled either during the transcription reaction itself using pre-labeled Nucleoside-triphosphates, or, alternatively, subsequent to the *in vitro* transcription by any kind of method known in the art, e.g. by using a ChemLink reagent.

Depending on the subsequent usage, a great variety of different labels available may be chosen, most of which are even commercially available. In a first aspect, the label may be a radioactive label like e.g. 32-P. in another aspect, the label may be a fluorescent label, e.g. Fluorescein, Red-640 (Roche Diagnostics), Cy5, Cy3 (Amersham), e. c.. In a further aspect, haptens like Digoxygenin (Roche Diagnostics) may be used, which can subsequently detected by an immunological reaction with an appropriate anti-DIG antibody. In a still further aspect, the label may be a hapten like e.g. Biotin which is detectable by means of interaction with Streptavidin (Roche Diagnostics).

As disclosed above, the invention is also directed to a method for expression profiling, wherein such a pool of labeled nucleic acids is hybridized to a plurality of nucleic acid probes, said probes being immobilized on a surface.. Usually, the plurality of nucleic acid probes are either oligonucleotides (Pease, A. C., et al., Proc Natl Acad Sci U S A (1994) 5022-6) or, alternatively, ds cDNA fragments e.g. PCR products (Schena, M., et al., Science (1995) 467-70).

For example, the plurality of nucleic acid probes may be immobilized on solid support. In this case, immobilization is achieved either by in-situ synthesis of the plurality of probes on the micoarray itself ((Pease, A. C., et al., Proc Natl Acad Sci U S A (1994) 5022-6) or, alternatively, by a sophisticated spotting technique (Cheung, V. G., et al., Nat Genet (1999) 15-9). Examples for different microarray platforms have already been disclosed in the art (Bowtell, D. D., Nat Genet (1999) 25-32) and, moreover are even already commercially available (Affymetrix, Perkin Elmer).

In a specific embodiment of expression profiling according to the invention, the labeled pool of nucleic acids is cut into fragments prior to hybridization. It has turned out to be advantageous, if the generated fragments are between 50 and 200 nucleotide residues in length. Depending on the nature of the labeled pool, fragmentation may be obtained by different means. If the labeled pool of nucleic acids consists of DNA, fragmentation can be obtained by limited DNase digestion, e.g. with DNaseI, or alternatively by subjecting the pool to sonication. If the labeled pool consists of RNA, a limited RNase digest, for example with RNaseA may be performed. Alternatively, the RNA may become thermally degraded, preferably by subjecting the probe to temperatures between 60 -70° C for 10 min - 2h.

The following examples, references, sequence listing and figures are provided to aid the understanding of the present invention, the true scope of which is set forth in the appended claims. It is understood that modifications can be made in the procedures set forth without departing from the spirit of the invention.

### Example 1: Isolation of total RNA from placenta tissue

Fresh, human Placenta tissue was stored in liquid Nitrogen for about 2-3 hours. Subsequently, total RNA was isolated with the RNeasy Maxi Kit (Qiagen) according to the manufacturer's animal tissue protocol.

First, the tissue was scrambled using pestle and pistil under liquid Nitrogen. To 0.6-0.8 mg scrambled tissue each, 15 ml RLT buffer + 150 µl β-Mercaptoethanol were added. Subsequently, homogenization was performed by passing the tissue lysate 6 times through a 0,80 x 120 mm needle and 6 times through a 0,60 x 80 mm needle fitted to a syringe. The tissue lysate was then centrifuged for 10 min at 5000 rpm (>8000 x g).

Afterwards, 1 Vol. 70 % ethanol was added to the supernatant and mixed immediately. 2 x 15 ml of this mixture were loaded on an RNeasy Maxi Column and centrifuged for 5 min at 5000 rpm (>8000 x g). Then, the column was washed with15 ml RW1 buffer and 10 ml RPE buffer. Total RNA was eluted with 0,8 ml RNase free water directly pipetted onto the RNeasy membrane. After incubation for 1 min at room temperature, the column was subsequently centrifuged for 1 min at 5000 rpm (>8000 x g). The elution steps were repeated in order to obtain 1,6 ml eluate. Total RNA was stored at -70°C.

### Example 2: cDNA Synthesis

### Step 1: first strand synthesis with a BOX T7 Oligo dT Primer

For the first strand synthesis, 50 ng total RNA from placenta tissue (total RNA concentration up to 1 µg is possible) were annealed with 50 pmol BOX T7 Oligo dT Primer( 5'GCATCATACAAGCTTGGTACCTGTAATACGACTCACTATAGGGAG GCGG(T)₂₄VN)(Seq. Id. No. 1) in a total volume of 10,5 µl for 10 min at 70°C. Then, the sample was rapidly cooled on ice and 9,5 µl Mastermix containing 4,0 µl RT-Puffer AMV 5x conc. (Roche Molecular Biochemicals); 2,0 µl DTT 0,1 M (Roche Molecular Biochemicals); 2,0 µl dNTP Mix 10 mM each (Roche Molecular Biochemicals); 0,5 µl RNase Inhibitor 40U/µl (Roche Molecular Biochemicals) and 1,0 µl AMV Reverse Transcriptase 25 U/µl (Roche Molecular Biochemicals) were added and mixed. Subsequently, the sample was incubated for 60 min at 42°C.

### Step 2: second strand synthesis with a BOX N10 Primer

Denaturation of the RNA/DNA Hybrid from step 1 was achieved through incubation for 5 min at 95°C followed by immediate cooling on ice.

For the second strand synthesis, 30 µl of a Mastermix containing 2,5 µl dNTP Mix 10 mM each; 5,0 µl BOXN10 Primer 100 pmol/µl (5'-GCATCATACAAGCTTGGTACC N10) (Seq. Id. No. 2), 5,0 µl Klenow Buffer 10x (500mM TRIS/HCl pH 7,5; 100mM MgCl2; 10 mM DTT); 13,5 µl RNase free water und 4,0 µl Klenow enzyme 2U/µl (Roche Molecular Biochemicals)) were added and mixed. Then the sample was incubated for 30 min at 37°C.

### Step 3: Purification of double stranded cDNA

This step was done using the High Pure PCR product Purification Kit (Roche Molecular Biochemicals).

As Carrier nucleic acid, 1,25 µl MSII RNA, (0,8 µg/µl, Roche Molecular Biochemicals) were added to the sample. RNase free water was added to a total volume of 100 µl.

Then, the sample was mixed with 500µl binding buffer, pipetted onto the High Pure Filtertube of the High Pure PCR product Purification Kit and centrifuged for 30 sec at 8000 rpm. Subsequently, the tube was washed with 1x 500µl and 1x 200µl washing buffer. Prior to elution, the filter tube was centrifuged to dryness for 2 min at 14000 rpm. For the elution, 50 µl elution buffer was added and the filter tube was centrifuged again for 1 min at 8000 rpm.

### Example 3: PCR - Determination of optimal cycle number

For the PCR reaction, two mastermixes were set up:

### Mastermix 1:

5.0 µl BOX Primer Primer (5'-GCATCATACAAGCTTGGTACC) (Seq. Id. No. 3, 10 µM); 2.0 µl dNTP Mix 10 mM each; 30.5 µl RNase free water.

### Mastermix 2:

10,0 µl Expand High Fidelity buffer 10x in 15 mM MgCl2 (Roche Molecular Biochemicals); 38.5 µl RNase free water and 1.5 µl Expand enzyme mix, High Fidelity 3.5 U/µl (Roche Molecular Biochemicals).

37.5 µl Mastermix 1 and 50 µl Mastermix 2 were added to 12,5 µl purified double stranded cDNA from example 2. PCR was performed in Perkin Elmer GeneAmp 9600 thermocycler according to the following protocol:

| | |
|---|---|
| Cycle number: | 30 |
| Denaturation: | 95°C 2 min |
| Denaturation | 95°C 30 sec (30x) |
| Annealing | 55°C 30 sec (30x) |
| Extension | 72°C 3 min (30x) |

Aliquots of 10 µl were removed after 18, 21, 24, 27 and 30 Cycles and analyzed on a 1,2 % agarose gel. A faint smear (indicative for an optimal cycle number) was observed after 24 cycles.

The PCR reaction was repeated for 24 cycles with a new sample. Purification of the amplification product was performed as described in step 3 of example 2 (see above).

### Example 4: in vitro transcription and labeling

*In vitro* transcription was performed using the following solutions:
Biotin labeling mix: 25 mM ATP (Roche Molecular Biochemicals), 25 mM CTP (Roche Molecular Biochemicals), 25 mM GTP (Roche Molecular Biochemicals), 18.75 mM UTP (Roche Molecular Biochemicals), 6,25 mM Biotin UTP (Roche Molecular Biochemicals).
T7 transcription mix: 200 ng purified PCR product; 4 µl Biotin labeling mix; 2 µl DTT 0,1 M, 2 µl 10x reaction buffer (Roche Molecular Biochemicals); 3 µl Enzyme mix (Roche Molecular Biochemicals); RNase free water ad 20 µl.
Samples of the T7 transcription mix were incubated for 3 hours at 37°C.
The Biotin labeled cRNA was subsequently purified using the High Pure RNA Tissue Kit (Roche Molecular Biochemicals) as follows: First, the sample was adjusted with RNase free Water to 100µl. Then, 400 µl binding buffer (including 10 µl β-Mercaptoethanol) and 200 µl ethanol absolute were added. After thorough mixing, the sample was pipetted onto the device of a High Pure RNA tissue purification kit . The device was then centrifuged for 15 sec at 8000 rpm. Washing was performed with 500 µl washing buffer 1, 500 µl washing buffer 2 and again 300 µl washing buffer 2, 1 min by 14000 rpm. Subsequently, the device was centrifuged to dryness for 1 min at 14000 rpm. Elution was performed using 2x 40 µl elution buffer and centrifugation for 1 min at 8000 rpm.

### Example 5: Gene Chip Analysis on Human Genome U95Av2 Array

Prior to hybridization, biotinylated human placenta cRNA prepared according to example 4 was fragmented for 35 min at 95°C. The fragmentation solution contained 15 µg RNA in 20 µ RNase free water and 5 µl fragmentation buffer (see Affimetrix GeneChip expression Analysis Manual).

A hybridization cocktail according to the Affimetrix GeneChip expression Analysis Manual was prepared, which included the biotinylated and fragmented cRNA (0,05 µg/µl), a control oligonucleotide B2 (50 pM), control cRNA Cocktail (BioB 1,5 pM, BioC 5 pM, BioD 25 pM and cre 100 pM respectively), salmon sperm DNA (0,1 mg/ml) (Promega), acetylated BSA (0,5 mg/ml) (Gibco); MES Hybridization Buffer (1x) and RNase free water to a final volume of 300 µl. The hybridization cocktail was heated to 99°C for 5 min, incubated 5 min at 45°C and spineed down for 5 min full speed thus rendering any insoluble matter at the bottom of the tube.

Prior to hybridization on a Human Genome U95Av2 array (Affimetrix), the probe array was filled with 200 µl MES Buffer (1x) and incubated at 45°C 10 min with a rotation of 60 rpm. After removal of the MES Buffer, the array cartridge was filled with 200 µl of the clarified hybridization cocktail avoiding any insoluble matter in the solution. Hybridization was performed for 16 h at 45°C in a rotisserie box with a rotation at 60 rpm.

Subsequently, the hybridization cocktail was removed and the probe array was filled with 250 µl non-stringent wash buffer (AffimetrixGeneChip expression Analysis Manual) and inserted in a GeneChip Fluidics Station 400. The fluidics program EukGE-WS2v2 was used for the subsequent washing and staining steps.

Staining was obtained by using the following solutions:
1^{st} stain: 300 µl 2xStain Buffer, 270 µl RNase free water, 24 µl of 50 mg/ml acetylated BSA and 6 µl of 1 mg/ml Strepatavidin, recombinant (Roche Molecular Biochemicals).
2^{nd} stain: 300 µl 2xStain Buffer, 266,4 µl RNase free water, 24 µl of 50 mg/ml acetylated BSA (Gibco), 6 µl of 10 mg/ml normal goat IgG (Sigma) and 3,6 µl of 0,5 mg/ml biotinylated anti streptavidin (Vector).
3^{rd} stain: 300 µl 2xStain Buffer, 270 µl RNase free water, 24 µl of 50 mg/ml acetylated BSA and 6 µl of 1 mg/ml SAPE (Molecular Probes).

After staining, the probe array was scanned with th HP GeneArray Scanner. Data were subsequently analyzed using the Affimetrix GeneChip software.

6100 positive genes were detected by starting with 50 ng of total human placenta RNA. This corresponds to 48% of the genes present on the Human Genome U95Av2 Gene Chip.

### Example 6: Reproducibility

From the same RNA isolated according to example 2, amplification according to example 4 and subsequent hybridization according to example 5 was performed in two independent experiments.

The correlation between the signal intensities obtained from the two different experiments was compared and analyzed be creating a scatter blot as shown in figure 2. The solid lines indicate a factor of 2 and 5 with regard to signal intensity differences . (It should be noted that data points below an intensity of 100 are at the detection limit and are highly variable.)

As it can be deduced from the figure, the results of the two independent experiments yielded comparable results, indicating the high reproducibility of the new method.

### List of References

Bowtell, D. D., Nat Genet (1999) 25-32
Cheung, V. G., et al., Nat Genet (1999) 15-9
Chomczynski, P. Sacchi, N., Anal Biochem (1987) 156-9
Kievits, T., et al., J Virol Methods (1991) 273-86
Kristjansson, K., et al., Nat Genet (1994) 19-23
Lavery, D. J., et al., Proc Natl Acad Sci U S A (1997) 6831-6
Matteucci, M. D. Caruthers, M. H., J. Am. Chem. Soc. (1981) 3185-3191
Milligan, J. F., et al., Nucleic Acids Res (1987) 8783-98
Money, T., et al., Nucleic Acids Res (1996) 2616-7
Nielsen, P. E., et al., Science (1991) 1497-500
Pease, A. C., et al., Proc Natl Acad Sci U S A (1994) 5022-6
Schena, M., et al., Science (1995) 467-70
Thibodeau, S. N., et al., Science (1993) 816-9
Welsh, J. McClelland, M., Nucleic Acids Res (1990) 7213-8
EP 0 957 177
US 5,262,311
US 5,962,272
WO 01/11082
WO 98/51789

Sequence listing

<110> Roche Diagnostics GmbH

F. HOFFMANN-LA ROCHE AG

<120> Improved method for random cDNA amplification

<130> 5506/00/EP

<140>

<141>

<160> 4

<170> PatentIn Ver. 2.1

<210> 1

<211> 75

<212> DNA

<213> Artificial Sequence

<220>

<223> Description of Artificial Sequence:primer

<400> 1

gcatcataca agcttggtac ctgtaatacg actcactata gggaggcggt tttttttttt 60

tttttttttt tttvn 75

<210> 2

<211> 31

<212> DNA

<213> Artificial Sequence

<220>

<223> Description of Artificial Sequence:primer

<400> 2

gcatcataca agcttggtac cnnnnnnnnn n                    31

<210> 3

<211> 21

<212> DNA

<213> Artificial Sequence

<220>

<223> Description of Artificial Sequence:primer

<400> 3

gcatcataca agcttggtac c                    21

<210> 4

<211> 47

<212> DNA

<213> Artificial Sequence

<220>

<223> Description of Artificial Sequence:primer

<400> 4

gcatcataca agcttggtac cttttttttt tttttttttt tttttvn 47

## Claims

1. Method for amplification of a pool of RNA sequences, comprising:
a) synthesis of total first strand cDNA with a first primer, said first primer being an extendible oligonucleotide comprising a first segment located at the 3' terminal part of said oligonucleotide which is capable of hybridizing to substantially all RNAs contained in the RNA pool and a second segment, said second segment beeing located more proximal to the 5' end of said oligonucleotide as compared to said first segment, said second segment being capable of serving as a primer binding side for another nucleic acid amplification primer by itself
b) synthesis of total second strand cDNA with a second primer, said second primer being an extendible oligonucleotide comprising a first randomized segment located at the 3' part of said primer and a second segment which is located more proximal to the 5' end of said oligonucleotide as compared to said first segment, said second segment being capable of serving as a primer binding site by itself for a nucleic acid amplification primer such that a double stranded cDNA is generated
c) amplification of said double stranded cDNA.

2. Method according to claim 1, wherein said first segment of said first primer is oligo-dT.

3. Method according to claims 1-2, wherein said pool of RNA sequences is either total cellular RNA or mRNA.

4. Method according to claims 1-3, wherein the randomized region of said second extendible primer is between 4 to 30 and preferably between 8- 12 nucleotides in length.

5. Method according to claims 1-4, wherein the first extendible primer additionally comprises a promotor for *in vitro* transcription, further comprising
d) *in vitro* transcription

6. Method according to claims 1-5, further comprising
e) repeating steps a - d)

7. Method according to claims 4-6, wherein a detectable label is incorporated either during or subsequent to the amplification reaction, or during or subsequent to the *in vitro* transcription reaction.

8. A pool of nucleic acids, labeled according to claim 7.

9. Method for expression profiling, wherein a pool of nucleic acids labeled according to claim 8 is hybridized to a plurality of nucleic acid probes, said probes being immobilized on a surface.

10. Method according to claim 9, wherein said pool of labeled nucleic acids is cut into fragments prior to hybridization, the generated fragments preferably being 50-200 nucleotide residues in length.
